# EUROPÄISCHE PATENTSCHRIFT

(11) **EP 3 004 048 B1**
(45) Veröffentlichungstag und Bekanntmachung des Hinweises auf die Patenterteilung: **08.03.2017**
(21) Anmeldenummer: 14725973.3
(22) Anmeldetag: 14.05.2014
(51) Int. Cl.: C07C 227/08

(54) **VERFAHREN ZUR HERSTELLUNG VON AMINOSÄUREN**
METHOD FOR PRODUCING AMINO ACIDS
PROCÉDÉ DE FABRICATION D'ACIDES AMINÉS

(30) Priorität: 24.05.2013 EP 13169152
(43) Veröffentlichungstag der Anmeldung: 13.04.2016
(73) Patentinhaber: BASF SE, 67056 Ludwigshafen am Rhein (DE)
(72) Erfinder: GRÜNANGER, Christian, 68163 Mannheim (DE); BOU CHEDID, Roland, 68159 Mannheim (DE); BIEL, Markus, Christian, 68159 Mannheim (DE); MELDER, Johann-Peter, 67459 Böhl-Iggelheim (DE)
(74) Vertreter: BASF IP Association
(86) Internationale Anmeldenummer: PCT/EP2014/059867
(87) Internationale Veröffentlichungsnummer: WO 2014/187712

(56) Entgegenhaltungen:
- NAKAMURA S ET AL: "Studies on the Formation of Amino Acids from Keto Acids", NIPPON NOGEI KAGAKUKAISHI - JOURNAL OF THE AGRICULTURAL CHEMICAL SOCIETY OF JAPAN, NIPPON NOGEI KAGAKUKAI, TOKYO, JP, Bd. 24, Nr. 4, 1. Januar 1950 (1950-01-01) , Seiten 185-187, XP008165506, ISSN: 0002-1407, DOI: 10.1271/NOGEIKAGAKU1924.24.185 [gefunden am 2008-11-21]
- WATKINS J C: "THE SYNTHESIS OF SOME ACIDIC AMINO ACIDS POSSESSING NEUROPHARMACOLOGICAL ACTIVITY", JOURNAL OF MEDICINAL AND PHARMACEUTICAL CHEMISTRY, AMERICAN CHEMICAL SOCIETY, EASTON, US, Bd. 91, 1. November 1962 (1962-11-01), Seiten 1187-1199, XP001156267, DOI: 10.1021/JM01241A010

## Beschreibung

Die vorliegende Erfindung betrifft ein Verfahren zur Herstellung von Alkalimetallsalzen von Glycin oder von racemischen α-Aminosäuren der allgemeinen Formel (I)

R¹-CH(NH₂)-COOH (I)

in denen R¹ gewählt wird aus Wasserstoff, CH₃, C₂H₅, CH(CH₃)₂, (CH₂)₂COOH und CH₂OH,
dadurch gekennzeichnet, dass man ein Alkalimetallsalz der korrespondierenden α-Ketocarbonsäure bzw. Glyoxalsäure in Gegenwart von mindestens einem heterogenen Katalysator, der mindestens ein Übergangsmetall enthält, in Gegenwart von Wasserstoff mit mindestens einer Stickstoffverbindung bei Temperaturen im Bereich von 50 bis 200°C umsetzt, wobei man Stickstoffverbindung wählt aus primären und sekundären Aminen und Ammoniak.

Aminosäuren haben zahlreiche Anwendungsgebiete. So werden L-Aminosäuren zur Peptid-und zur Proteinsynthese eingesetzt. Doch auch racemische Aminosäuren sind begehrte Zwischenprodukte.

Die Herstellung von racemischen Aminosäuren durch die Streckersynthese ist an sich bekannt. Nachteilig ist, dass man mit Blausäure bzw. den entsprechenden Cyaniden sehr toxische Substanzen benötigt, die besondere Sicherheitsvorkehrungen notwendig machen.

Aus US 2004/092725 ist ein Verfahren bekannt, α-Aminosäuren aus korrespondierenden α-Hydroxycarbonsäuren zu synthetisieren, indem man mit Ammoniak bei hohem Druck und vorzugsweise mindestens 300°C umsetzt. Die Ausbeuten sind allerdings gering. So wird die Synthese von Glycin bei 374°C mit einer Ausbeute von 4,3% und die Synthese von α-Alanin bei 374°C mit einer Ausbeute von 2,8% offenbart. Derartige Ausbeuten sind in großtechnischen Verfahren allerdings unbefriedigend. Außerdem sind die erhaltenen Produkte meist dunkel verfärbt und erfordern dann ein aufwändiges Reinigungsverfahren.

J. Med. Pharm. Chem. 1962, 91, 1187 offenbart ein Verfahren zur Herstellung von racemischen α-Aminosäuren durch Hydrogenierung von α-Ketoglutaräure in Methylamin in Anwesenheit von Raney-Nickel. Die Ausbeute ist 40%. Die Autoren schlagen eine Aufreinigung durch Säulenchromatographie mit wässrigem Pyridin als Laufmittel vor. Ein derartiges Verfahren ist im großtechnischen Maßstab unattraktiv.

J. Agricultural Soc. Japan, 1950, 24, 185 offenbart die Umsetzung einer freien Ketosäure oder ihres Ammoniumsalzes zu einer Aminosäure.

Aus US 2013/0012737 ist ein Verfahren zur Herstellung des Natriumsalzes von 2-Amino-4-methylthiobuttersäure bekannt, in dem Salze der entsprechenden Ketosäuren reduzierend aminiert werden.

Andere α-Ketosäuren zersetzen sich allgemein jedoch recht leicht, v. a. bei höheren Temperaturen und scheinen daher als Vorstufe zur Herstellung von racemischen Aminosäuren nicht geeignet.

Es bestand also die Aufgabe, ein Verfahren bereit zu stellen, durch das man racemische α-Aminosäuren in guten Ausbeuten gewinnen kann.

Dementsprechend wurde das eingangs definierte Verfahren gefunden, im Rahmen der vorliegenden Erfindung auch erfindungsgemäßes Verfahren genannt.

Im Falle der Herstellung von Glycin, welches nicht chiral ist, erhält man kein Racemat, sondern Glycin.

Im Rahmen der vorliegenden Erfindung wird Glyoxalsäure auch als α-Ketocarbonsäure der allgemeinen Formel (II) mit R¹ = H betrachtet.

Zur Durchführung des erfindungsgemäßen Verfahrens geht man aus von mindestens einer korrespondierenden α-Ketocarbonsäure, also von einer α-Ketocarbonsäure der allgemeinen Formel (II),

R¹-C(=O)-COOH (II)

in denen R¹ gewählt wird aus Wasserstoff, CH₃, C₂H₅, CH(CH₃)₂, (CH₂)₂COOH und CH₂OH, bevorzugt ist R¹ gleich CH₃;
beispielsweise von einem Gemisch von zwei oder drei α-Ketocarbonsäuren der allgemeinen Formel (II). Bevorzugt setzt man genau eine α-Ketocarbonsäure der allgemeinen Formel (II) ein.

Bevorzugte Beispiele für α-Ketocarbonsäuren der allgemeinen Formel (II) sind Glyoxalsäure, 2-Oxoglutarsäure, 3-Hydroxy-2-Oxopropionsäure, 2-Oxo-4-Methylbuttersäure und insbesondere Brenztraubensäure.

α-Ketocarbonsäure der allgemeinen Formel (II) setzt man in partiell oder vollständig neutralisierter Form ein, vorzugsweise in vollständig neutralisierter Form, und zwar als Alkalimetallsalz wie beispielsweise als Kaliumsalz und bevorzugt als Natriumsalze. Ganz besonders bevorzugt setzt man α-Ketocarbonsäure der allgemeinen Formel (II) mit einem Überschuss Base ein, wobei man Base wählen kann aus Alkalimetall(hydrogen)carbonat und Alkalimetallhydroxid, beispielsweise Natriumhydrogencarbonat, Kaliumhydrogencarbonat, Natriumcarbonat, Kaliumcarbonat, Kaliumhydroxid und insbesondere Natriumhydroxid.

In einer Ausführungsform der vorliegenden Erfindung führt man das erfindungsgemäße Verfahren in wässrigem Milieu durch. Darunter ist zu verstehen, dass man Alkalimetallsalz von α-Ketocarbonsäure der allgemeinen Formel (II) in Wasser löst oder in einem Gemisch, das zu mindestens 75 Vol.-% Wasser enthält und insgesamt bis zu 25 Vol.-% organisches Lösungsmittel enthalten kann, beispielsweise Tetrahydrofuran oder N,N-Dimethylformamid, wobei Vol.-% auf gesamte kontinuierliche Phase bezogen ist. Vorzugsweise setzt man kein organisches Lösungsmittel oder nur 0,1 bis 5 Vol.-% organisches Lösungsmittel ein, bezogen auf kontinuierliche Phase. In einer anderen Variante kann man α-Ketocarbonsäure der allgemeinen Formel (II) in Wasser oder in einem Gemisch, das zu mindestens 75 Vol.-% Wasser enthält und insgesamt bis zu 25 Vol.-% organisches Lösungsmittel enthalten kann, beispielsweise Tetrahydrofuran oder N,N-Dimethylformamid, lösen oder suspendieren und dann mit vorzugsweise wässriger Lösung von Alkalimetall(hydrogen)carbonat und Alkalimetallhydroxid zumindest partiell und vorzugsweise vollständig neutralisieren.

In einer Ausführungsform der vorliegenden Erfindung führt man das erfindungsgemäße Verfahren bei einem pH-Wert im Bereich von 6 bis 14 durch, besonders bevorzugt 7 bis 13,5.

Erfindungsgemäß setzt man Alkalimetallsalz von α-Ketocarbonsäure der allgemeinen Formel (II) mit mindestens einer Stickstoffverbindung um, wobei man Stickstoffverbindung wählt aus primären Aminen, bevorzugt sekundären Aminen und noch mehr bevorzugt Ammoniak. Beispiele für primäre Amine sind insbesondere C₁-C₁₀-Alkylamine, beispielsweise Methylamin, Ethylamin, Isopropylamin, tert.-Butylamin, n-Decylamin, weiterhin aromatische Amine wie Anilin, C₃-C₇-Cycloalkylamine wie beispielsweise Cyclohexylamin und Monoethanolamin. Beispiele für sekundäre Amine sind Di-C₁-C₁₀-Alkylamine, insbesondere Dimethylamin, Diethylamin und Diisopropylamin, weiterhin Di-C₂-C₄-hydroxyalkylenamine, insbesondere Diethanolamin, weiterhin Mono-C₁-C₁₀-alkylmono-C₂-C₄-hydroxyalkylenamine, beispielsweise N-Methyl-N-ethanolamin, weiterhin cyclische sekundäre Amine wie Piperidin und Morpholin. Weitere geeignete Stickstoffverbindungen sind Iminodicarbonsäuren, insbesondere Iminodiessigsäure.

In einer bevorzugten Ausführungsform der vorliegenden Erfindung wählt man als Stickstoffverbindung Ammoniak, das heißt man setzt mit Ammoniak um. Ammoniak kann man der Reaktionsmischung in Form von flüssigem Ammoniak, gasförmigem Ammoniak oder Ammoniakwasser ("NH₄OH") zusetzen. Wünscht man Ammoniak in Form eines Ammoniumsalzes zuzusetzen, so ist es bevorzugt, Ammoniak in Kombination mit mindestens einer starken Base zuzusetzen, beispielsweise in Kombination mit NaOH oder KOH. Bevorzugt ist es, Ammoniak in Form von flüssigem Ammoniak oder Ammoniakwasser zuzusetzen.

In einer Ausführungsform setzt man α-Ketocarbonsäure der allgemeinen Formel (II) und Stickstoffverbindung in einem Molverhältnis im Bereich von 1 : 1 bis 1 : 100 ein, bevorzugt im Bereich von 1 : 2 bis 1 : 50, besonders bevorzugt im Bereich von 1 : 3 bis 1 : 30. Dabei bezieht sich der Anteil an Stickstoffverbindung auf die Summe aller als Reaktionspartner eingesetzten Stickstoffverbindungen.

In einer Ausführungsform setzt man α-Ketocarbonsäure der allgemeinen Formel (II) und Ammoniak in einem Molverhältnis im Bereich von 1 : 1 bis 1 : 100 ein, bevorzugt im Bereich von 1 : 2 bis 1 : 50, besonders bevorzugt im Bereich von 1 : 3 bis 1 : 30.

Das erfindungsgemäße Verfahren führt man in Gegenwart von Wasserstoff durch, also von H₂. In einer Ausführungsform der vorliegenden Erfindung führt man das erfindungsgemäße Verfahren so durch, dass man insgesamt ein Molverhältnis α-Ketocarbonsäure der allgemeinen Formel (II) zu Wasserstoff im Bereich von 1:1 bis 1:90, bevorzugt 1:2 bis 1:30 einstellt.

In einer Ausführungsform der vorliegenden Erfindung kann man Wasserstoff durch ein unter den Reaktionsbedingungen des erfindungsgemäßen Verfahrens inertes Gas verdünnen, beispielsweise mit Stickstoff oder mit mindestens einem Edelgas, beispielsweise mit Argon.

Das erfindungsgemäße Verfahren führt man in Gegenwart von mindestens einem heterogenen Katalysator durch, der mindestens ein Übergangsmetall enthält. Als heterogene Katalysatoren können dabei dienen:
(i) auf einem festen Träger, der in partikulärer Form vorliegt, geträgerte Metall-haltige Katalysatoren,
(ii) auf einem festem Träger, der in nicht-partikulärer Form vorliegt, geträgerte Metall-haltige Katalysatoren,
(iii) Träger-freie katalytisch aktive Partikel.

Im Rahmen der vorliegenden Erfindung umfasst der Begriff Katalysator dabei Übergangsmetall, das als katalytisch aktive Spezies ("Haupt-Metall") dient, oder gegebenenfalls eine Vorstufe davon, weiterhin gegebenenfalls vorhandener Träger und gegebenenfalls vorhandene Dotierung.

Unter "festem Träger" sollen dabei solche Materialien verstanden werden, die unter den Reaktionsbedingungen des erfindungsgemäßen Verfahrens fest sind und die für die Formgebung des heterogenen Katalysators maßgeblich sind.

Unter "in partikulärer Form vorliegend" soll verstanden werden, dass der betreffende Träger in Form von Partikeln vorliegt, deren durchschnittlicher Durchmesser im Bereich von 0,1 µm bis 2 mm liegt, bevorzugt 0,001 bis 1 mm, besonders bevorzugt im Bereich von 0,005 bis 0,5 mm, insbesondere 0,01 bis 0,25 mm.

Unter "in nicht-partikulärer Form vorliegend" soll verstanden werden, dass der Träger in mindestens einer Dimension (Breite, Höhe, Tiefe) mehr als 2 mm, bevorzugt mindestens 5 mm aufweist, wobei mindestens eine weitere Dimension, beispielsweise ein oder beide weiteren Dimensionen, kleiner als 2 mm groß sein kann, beispielsweise im Bereich von 0,1 µm bis 2 mm. In einer anderen Variante weist in nicht-partikulärer Form vorliegender Träger drei Dimensionen auf, die eine Abmessung von mehr als 2 mm, bevorzugt mindestens 5 mm, aufweisen. Als Obergrenze sind beispielsweise 10 m, bevorzugt 10 cm geeignet. Beispiele für Träger, die in nicht-partikulärer Form vorliegen, sind Metallnetze, beispielsweise Stahlnetze oder Nickelnetze, weiterhin Drähte wie Stahldrähte oder Nickeldrähte, weiterhin Formkörper, beispielsweise Kugeln, Raschig-Ringe, Stränge und Tabletten.

In einer Ausführungsform der vorliegenden Erfindung setzt man Katalysator in Form von Formkörpern ein, beispielsweise in Form von Tabletten oder Strängen.

Beispiele für besonders geeignete Dimensionen von Formkörpern sind Tabletten mit Abmessungen 6.3 mm, 3.3 mm, 2.2 mm, und Stränge mit einem Durchmesser im Bereich von 1,5 bis 3 mm.

Beispiele für Träger, die in partikulärer Form vorliegen, sind Pulver, die frei fließend oder suspendiert sein können.

Beispiele für Materialien, aus denen Träger, die in partikulärer Form vorliegen, sein können, sind Al₂O₃, SiO₂, Alumosilikate, Hydrotalcit, TiO₂, ZrO₂, Aktivkohle, insbesondere Al₂O₃, ZrO₂, und TiO₂.

Beispiele für Träger-freie katalytisch aktive Partikel (iii) sind Raney-Metalle, beispielsweise Raney-Kupfer, Raney-Nickel und Raney-Kobalt. Träger-freie katalytisch aktive Partikel können beispielsweise als Schwamm- oder Skelett-Katalysatoren vorliegen.

Katalysator kann neben Träger und Übergangsmetall ein oder mehrere Formmittel enthalten, beispielsweise Graphit oder Stearinsäure.

Beispiele für Übergangsmetalle, die als katalytisch aktive Spezies ("Haupt-Metall") in metallhaltigem Katalysator für das erfindungsgemäße Verfahren geeignet sind, sind Übergangsmetalle der Gruppen 4 bis 12 des Periodensystems, und zwar bevorzugt Übergangsmetalle der ersten Periode der Gruppen 4 bis 12 des Periodensystems, also von Ti bis Zn, sowie Übergangsmetalle der Gruppen 8 bis 11 sämtlicher Perioden des Periodensystems. Besonders bevorzugte Übergangsmetalle sind Co, Ni und Cu, weiterhin Pd und Pt.

Übergangsmetall in Katalysator, der für das erfindungsgemäße Verfahren eingesetzt wird, kann dotiert sein, beispielsweise mit einem oder mehreren anderen Übergangsmetallen wie Zr, Mo, Mn oder Ti, oder mit Ca, mit Sn, mit Al oder mit Na. Unter Dotieren sollen hierbei Mengen an dotiertem Übergangsmetall bzw. Na, Al oder Ca verstanden werden, die man im Bereich von 0,1 bis 2 mol-% Übergangsmetall bzw. Na, Sn, Al oder Ca, bezogen auf Haupt-Metall, einbringt. Im Rahmen der vorliegenden Erfindung gelten jedoch aus der Gewinnung von Hauptmetall herrührende übliche Begleit-Spurenelemente als von einer Dotierung ausgenommen.

In einer Ausführungsform der vorliegenden Erfindung wählt man heterogene Katalysatoren aus Raney-Metallen und auf einem festen Träger aufgebrachtem Übergangsmetall. Bevorzugtes Übergangsmetall (Haupt-Metall) ist gewählt aus Ni, Cu und Co.

Zur Herstellung eines für das erfindungsgemäße Verfahren geeigneten Katalysators und zu seiner Lagerung setzt man Übergangsmetall in der Regel als Vorstufe, ein, nämlich als Verbindung, beispielsweise als Oxid, Hydroxid oder Oxidhydroxid, oder als Legierung, und aktiviert den Katalysator vor der Durchführung des erfindungsgemäßen Verfahrens oder *in situ*, vorzugsweise durch Reduktion oder durch Entfernung von mindestens einer Komponente der betreffenden Legierung. Vorzugsweise liegt Übergangsmetall in heterogenem Katalysator während der Durchführung des erfindungsgemäßen Verfahrens anteilig zumindest zeitweise in der Oxidationsstufe null vor.

In einer Ausführungsform der vorliegenden Erfindung wählt man Katalysatoren aus solchen Materialien in partikulärer Form, deren Masse - jeweils bestimmt vor der Aktivierung mit Wasserstoff - enthält:
insgesamt im Bereich von 15 bis 80 Gew.-% sauerstoffhaltige Verbindung(en) des Aluminiums, berechnet als Al₂O₃, bevorzugt 30 bis 70 Gew.-%, besonders bevorzugt 35 bis 65 Gew.-%,
insgesamt im Bereich von 5 bis 35 Gew.-% sauerstoffhaltige Verbindung(en) des Nickels, berechnet als NiO, bevorzugt im Bereich von 10 bis 30 Gew.-%, besonders bevorzugt im Bereich von 12 bis 28 Gew.-%, ganz besonders bevorzugt 15 bis 25 Gew.-%,
insgesamt im Bereich von 5 bis 35 Gew.-% sauerstoffhaltige Verbindung(en) des Kobalts, berechnet als CoO, bevorzugt im Bereich von 10 bis 30 Gew.-%, besonders bevorzugt im Bereich von 12 bis 28 Gew.-%, ganz besonders bevorzugt 15 bis 25 Gew.-%,
insgesamt im Bereich von 1 bis 20 Gew.-% sauerstoffhaltige Verbindung(en) des Kupfers, berechnet als CuO, bevorzugt 2 bis 18 Gew.-%, besonders bevorzugt 5 bis 15 Gew.-%, insgesamt im Bereich von 0,2 bis 5 Gew.-% sauerstoffhaltige Verbindung(en) des Zinns, berechnet als SnO, bevorzugt im Bereich von 0,4 bis 4,0 Gew.-%, besonders bevorzugt im Bereich von 0,6 bis 3,0 Gew.-%, ganz besonders bevorzugt im Bereich von 0,7 bis 2,5 Gew.-%.

In einer Ausführungsform der vorliegenden Erfindung wählt man Katalysatoren aus solchen Materialien in partikulärer Form, deren Masse - jeweils bestimmt vor der Aktivierung mit Wasserstoff - enthält:
22 bis 45 Gew.-%, bevorzugt 25 bis 40 Gew.-%, sauerstoffhaltige Verbindungen des Zirkoniums, berechnet als ZrO₂,
1 bis 30 Gew.-%, bevorzugt 2 bis 25 Gew.-%, besonders bevorzugt 5 bis 15 Gew.-%, sauerstoffhaltige Verbindungen des Kupfers, berechnet als CuO,
5 bis 50 Gew.-%, bevorzugt 15 bis 45 Gew.-%, besonders bevorzugt 25 bis 40 Gew.-%, sauerstoffhaltige Verbindungen des Nickels, berechnet als NiO,
5 bis 50 Gew.-% sauerstoffhaltige Verbindungen des Kobalts, berechnet als CoO,
0 bis 10 Gew.-% sauerstoffhaltige Verbindungen des Aluminiums und/oder Mangans, wobei das Gewichtsverhältnis von Zirkonium, berechnet als ZrO₂, zu Aluminium und/oder Mangan, berechnet als Al₂O₃ und/oder MnO₂, bevorzugt mindestens 2,5 beträgt, ganz besonders bevorzugt 0 Gew.-% sauerstoffhaltige Verbindungen des Aluminiums und/oder Mangans, 0 bis 5 Gew.-%, bevorzugt 0 Gew.-% sauerstoffhaltige Verbindungen des Molybdäns, berechnet als MoO₃.

In einer Ausführungsform der vorliegenden Erfindung wählt man Katalysatoren aus solchen Materialien in partikulärer Form, deren Masse - jeweils bestimmt vor der Aktivierung mit Wasserstoff - enthält:
50 bis 95 Gew.-%, bevorzugt 55 bis 85 Gew.-%, besonders bevorzugt 60 bis 80 Gew.-% sauerstoffhaltige Verbindungen des Zirkoniums, berechnet als ZrO₂,
5 bis 50 Gew.-%, bevorzugt 15 bis 45 Gew.-%, besonders bevorzugt 20 bis 40 Gew.-%, sauerstoffhaltige Verbindungen des Nickels, berechnet als NiO.

In einer Ausführungsform der vorliegenden Erfindung beträgt das Molverhältnis von Nickel zu Kupfer größer 1, besonders bevorzugt größer 1,2, ganz besonders bevorzugt im Bereich von 1,8 bis 8,5.

Bevorzugt enthält die katalytisch aktive Masse von im erfindungsgemäßen Verfahren eingesetzten Katalysator kein Rhenium, kein Ruthenium, kein Eisen und/oder kein Zink, jeweils weder in metallischer (Oxidationsstufe = 0) noch in ionischer (Oxidationsstufe # 0).

In einer Ausführungsform der vorliegenden Erfindung hat für erfindungsgemäßes Verfahren geeigneter Katalysator eine BET-Oberfläche im Bereich von 1 bis 1000 m²/g, bevorzugt von 10 bis 500 m²/g, gemessen durch N₂-Adsorption nach DIN 66131.

Zur Herstellung von bevorzugten im erfindungsgemäßen Verfahren verwendeten Katalysatoren der Variante (i) und (ii) sind verschiedene Verfahren möglich. Geeignete Katalysatoren der Variante (i) und (ii) sind beispielsweise durch Verkneten pulvriger Mischungen der Hydroxide, Carbonate, Oxide und/oder anderer Salze der Komponenten mit Wasser und nachfolgendes Extrudieren und Tempern (Wärmebehandlung) der so erhaltenen Masse erhältlich.

Bevorzugt werden zur Herstellung der im erfindungsgemäßen Verfahren eingesetzten Katalysatoren der Variante (i) und (ii) Fällungsmethoden angewandt. So können bevorzugte Katalysatoren beispielsweise durch eine gemeinsame Fällung von Nickel-, Kobalt-, Kupfer-und Zinn-Komponenten aus einer diese Elemente enthaltenden wässrigen Salzlösung mittels Basen in Gegenwart einer Aufschlämmung einer schwerlöslichen, sauerstoffhaltigen Aluminium-, Titan-, Silizium- und/oder Zirkoniumverbindung (Präzipitat) und anschließendes Waschen, Trocknen und Calcinieren des erhaltenen Präzipitats erhalten werden. Als schwerlösliche, sauerstoffhaltige Aluminium-, Titan-, Silizium- und/oder Zirkoniumverbindungen können beispielsweise deren Oxide, Oxidhydrate, Phosphate, - borate und -silikate Verwendung finden. Aufschlämmungen der schwerlöslichen sauerstoffhaltigen Aluminium-, Titan-, Silizium- und/oder Zirkoniumverbindungen kann man durch Suspendieren feinkörniger Pulver derartiger Verbindungen in Wasser unter kräftigem Rühren herstellen. Bevorzugt stellt man Aufschlämmungen von schwerlöslichen sauerstoffhaltigen Aluminium-, Titan-, Silizium- bzw. Zirkoniumverbindungen durch Ausfällen der entsprechenden schwerlöslichen sauerstoffhaltigen Aluminium-, Titan-, Silizium- und Zirkoniumverbindungen aus wässrigen Lösungen von Aluminium-, Titan-, Silizium- bzw. Zirkoniumverbindungen mittels Base her.

Bevorzugt werden im erfindungsgemäßen Verfahren eingesetzte Katalysatoren der Variante (i) und (ii) über eine gemeinsame Fällung (Mischfällung) aller ihrer Komponenten hergestellt. Dazu wird zweckmäßigerweise eine die Katalysatorkomponenten enthaltende, wässrige Salzlösung in der Wärme und unter Rühren so lange mit einer wässrigen Base-beispielsweise Natriumcarbonat, Natriumhydroxid, Kaliumcarbonat oder Kaliumhydroxid-versetzt, bis die Fällung vollständig ist. Es kann auch mit Alkalimetall-freien Basen wie Ammoniak, Ammoniumcarbonat, Ammoniumhydrogencarbonat, Ammoniumcarbamat, Ammoniumoxalat, Ammoniummalonat, Urotropin, Harnstoff, etc. gearbeitet werden. Die Art der verwendeten Salze ist im Allgemeinen nicht kritisch: Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zu Störungen führen, sei es, indem sie unerwünschte Fällungen verursachen oder indem sie durch Komplexbildung die Fällung erschweren oder verhindern.

Die bei diesen Fällungsreaktionen erhaltenen Niederschläge sind im Allgemeinen chemisch uneinheitlich und bestehen u.a. aus Mischungen der Oxide, Oxidhydrate, Hydroxide, Carbonate und unlöslichen und basischen Salze der eingesetzten Metalle. Es kann sich für die Filtrierbarkeit der Niederschläge als günstig erweisen, wenn sie gealtert werden, d. h. wenn man sie noch einige Zeit nach der Fällung, gegebenenfalls in Wärme oder unter Durchleiten von Luft, sich selbst überlässt.

Die nach Fällungsverfahren erhaltenen Niederschläge kann man zu im erfindungsgemäßen Verfahren eingesetzten Katalysatoren der Variante (i) und (ii) nach an sich bekannten Methoden weiterverarbeiten. Zunächst wäscht man die Niederschläge. Über die Dauer des Waschens und über die Temperatur und Menge des Waschwassers kann der Gehalt an Alkalimetall, das durch die als Fällungsmittel eventuell verwendete (Mineral)base zugeführt wurde, beeinflusst werden. Im Allgemeinen wird durch Verlängerung des Waschens oder Erhöhung der Temperatur des Waschwassers der Gehalt an Alkalimetall abnehmen. Nach dem Waschen kann man die Niederschläge im Allgemeinen bei 80 bis 200 °C, vorzugsweise bei 100 bis 150 °C, trocknen und danach calcinieren. Das Calcinieren kann man bei Temperaturen zwischen 300 und 800 °C, vorzugsweise bei 400 bis 600 °C, insbesondere bei 420 bis 550 °C ausführen.

In einer anderen Ausführungsform kann man im erfindungsgemäßen Verfahren eingesetzten Katalysatoren der Variante (i) und (ii) durch Tränkung (Imprägnieren) von Aluminiumoxid (Al₂O₃), Titandioxid (TiO₂), Siliziumdioxid (SiO₂) oder Zirkoniumdioxid (ZrO₂), das jeweils beispielsweise in Form von Pulver oder Formkörpern, wie Strängen, Tabletten, Kugeln oder Ringen, vorliegt, oder von Mischungen von mindestens zwei der vorstehend genannten Oxide mit Übergangsmetallsalzösung herstellen.

Aluminiumoxid wird beispielsweise in der amorphen, gamma-, theta- und/oder delta- Form, als Aluminiumoxohydroxid (Böhmit), bevorzugt in der gamma-Form eingesetzt.

Zirkoniumdioxid kann man beispielsweise in amorpher, monokliner, tetragonaler oder kubischer Modifikation einsetzen, bevorzugt sind die monokline, die tetragonale und die kubische Modifikation. Besonders bevorzugt ist die monokline Modifikation.

Die Herstellung von Formkörpern kann nach an sich bekannten Methoden erfolgen.

In einer Ausführungsform der vorliegenden Erfindung setzt man 0,1 bis 120 Gew.-% Katalysator ein, bezogen auf α-Ketocarbonsäure der allgemeinen Formel (II).

Das erfindungsgemäße Verfahren führt man bei einer Temperatur im Bereich von 50 bis 200°C, bevorzugt 75 bis 175°C und besonders bevorzugt von 100 bis 150°C durch.

In einer Ausführungsform des erfindungsgemäßen Verfahrens führt man es bei einem Druck im Bereich von Normaldruck bis 300 bar, bevorzugt 20 bis 250 bar, besonders bevorzugt von 100 bis 200 bar durch.

Das erfindungsgemäße Verfahren kann man absatzweise, kontinuierlich oder semi-kontinuierlich durchführen.

In einer Ausführungsform der vorliegenden Erfindung kann man die gesamte Reaktionsmischung oder bestimmte Komponenten der Reaktionsmischung im Kreis führen, beispielsweise Stickstoffverbindung, insbesondere Ammoniak, oder wässrige Lösung von α-Ketocarbonsäure der allgemeinen Formel (II).

In einer Ausführungsform der vorliegenden Erfindung führt man das erfindungsgemäße Verfahren absatzweise bei im Wesentlichen konstanter Temperatur durch, beispielsweise bei einer Temperatur, die während des erfindungsgemäßen Verfahrens um 10°C oder weniger schwankt, vorzugsweise um 5°C oder weniger.

In einer Ausführungsform der vorliegenden Erfindung führt man das erfindungsgemäße Verfahren über eine Dauer im Bereich von 1 Minute bis 48 Stunden durch. Wenn man das erfindungsgemäße Verfahren kontinuierlich durchzuführen wünscht, so soll unter der Dauer die mittlere Verweilzeit verstanden werden.

In einer Ausführungsform der vorliegenden Erfindung führt man das erfindungsgemäße Verfahren als Suspension von mindestens einem Katalysator nach Variante (i) oder (iii) durch, und zwar mit einer Reaktionsdauer im Bereich von 1 bis 48 Stunden, bevorzugt 2 bis 24 Stunden.

In einer anderen Ausführungsform der vorliegenden Erfindung führt man das erfindungsgemäße Verfahren mit einem Katalysator gemäß Variante (ii) durch, und zwar mit einer Reaktionsdauer im Bereich von 1 Minute bis 10 Stunden, bevorzugt 30 Minuten bis 5 Stunden.

Bei der Berechnung der Zeit sollen Zeiträume, die man zu Tätigkeiten wie Aufheizen, Abkühlen, Aufarbeitung der Reaktionsmischung, Isolierung von racemischer α-Aminosäure, Entspannen oder Aktivieren des Katalysators verwendet, nicht mit berücksichtigt werden.

In einer Ausführungsform der vorliegenden Erfindung kann man zur Durchführung des erfindungsgemäßen Verfahrens mischen, beispielsweise durch Rühren, Schütteln, Wälzen, Umpumpen, Pumpen durch statische Mischer oder pneumatisches Mischen.

Ohne dass einer bestimmten Theorie der Vorzug gegeben werden soll, so wird vermutet, dass im Verlaufe des erfindungsgemäßen Verfahrens α-Ketocarbonsäure zur entsprechenden α-Iminocarbonsäure umgewandelt und dann zu racemischer α-Aminocarbonsäure reduziert wird.

Man erhält eine Reaktionsmischung, die Wasser und Alkalimetallsalz von racemischer α-Aminosäure enthält und weitere Bestandteile aufweisen kann, beispielsweise Katalysator(reste), Ausgangsmaterial wie α-Ketocarbonsäure oder Stickstoffverbindung , insbesondere Ammoniak, oder weiterhin Zersetzungsprodukte von α-Ketocarbonsäure, beispielsweise Propionsäure, Essigsäure oder Ameisensäure. Bevorzugt enthält die Reaktionsmischung jedoch nur äußerst geringe Anteile an Zersetzungsprodukte(n) von α-Ketocarbonsäure.

In einer Ausführungsform der vorliegenden Erfindung arbeitet man die entstehende Reaktionsmischung auf. In einer speziellen Ausführungsform der vorliegenden Erfindung isoliert man racemische α-Aminosäure bzw. ein Salz von racemischer α-Aminosäure.

Zur Aufarbeitung kann man beispielsweise eine oder mehrere folgenden Tätigkeiten ausführen:
(i) Katalysator deaktivieren,
(ii) Katalysator, der aktiv oder deaktiviert sein kann, abtrennen, beispielsweise durch Filtrieren, beispielsweise Kuchenfiltration oder Querstromfiltration, oder durch Sedimentation oder Zentrifugieren,
(iii) Wasser und Stickstoffverbindung , insbesondere Ammoniak, ganz oder teilweise entfernen, beispielsweise durch Verdampfen, Abdestillieren oder Sprühtrocknen,
(iv) Stickstoffverbindung oder insbesondere Ammoniak mit Säure neutralisieren, insbesondere mit Brönsted-Säure, beispielsweise mit Schwefelsäure oder Salzsäure,
(v) Nebenprodukte abtrennen, die beispielsweise durch Reduktion von eingesetzter α-Ketocarbonsäure entstehen können,
(vi) pH-Wert einstellen, beispielsweise mit Brönsted-Säure oder Brönsted-Base,
(vii) α-Aminosäure von nicht umgesetzter α-Ketocarbonsäure mittels Ionenaustauscher abtrennen.

In einer Ausführungsform der vorliegenden Erfindung kristallisiert man anfallende racemische α-Aminosäure bzw. Salz von racemischer α-Aminosäure zur Aufreinigung um. Zum Umkristallisieren kann man verschiedene Lösungsmittel verwenden. Geeignet sind beispielsweise Wasser und wasserhaltige Gemische, beispielsweise Gemische von Wasser mit Ethanol. Bevorzugt kristallisiert man aus Wasser oder wässrigen Basen um, die einen pH-Wert im Bereich von 7,1 bis 14 aufweisen, bevorzugt 9 bis 12. Geeignete wässrige Basen sind verdünnte Kalilauge und insbesondere verdünnte Natronlauge.

Man kann ein- oder mehrmals umkristallisieren. Kristallisierte racemische α-Aminosäure bzw. kristallisiertes Alkalimetallsalz von racemischer α-Aminosäure kann man von der Mutterlauge abtrennen, beispielsweise durch Dekantieren oder Filtrieren oder Kombination von Filtrieren und Dekantieren.

In einer Ausführungsform erhält man reine racemische α-Aminosäure bzw. reines Salz von racemischer α-Aminosäure, beispielsweise reines Natriumsalz oder reines Kaliumsalz, oder partiell neutralisierte reine racemische α-Aminosäure.

Der Nachweis, dass es sich bei racemischer α-Aminosäure (a) um das Racemat handelt, gelingt beispielsweise durch Polarimetrie.

Nach dem erfindungsgemäßen Verfahren hergestellte Alkalimetallsalze von racemischen α-Aminosäuren der allgemeinen Formel (I)

R¹-CH(NH₂)-COOH (I)

in denen R¹ gewählt wird aus Wasserstoff, CH₃, C₂H₅, CH(CH₃)₂, (CH₂)₂COOH und CH₂OH, kann man vorzüglich einsetzen, um beispielsweise Komplexbildner zu machen, beispielsweise Nitrilotriessigsäure (mit R¹ = Wasserstoff), Methylglycindiessigsäure (mit R¹ = CH₃), oder Glutaminsäurediessigsäure (mit R¹ = (CH₂)₂COOH).

Die Erfindung wird durch die folgenden Arbeitsbeispiele weiter erläutert.
I. Herstellung des Natriumsalzes von racemischem Alanin 50 g einer 60 Gew.-% wässrigen Lösung des Natrium-Salzes der Brenztraubensäure (entsprechend 0,27 mol Natriumpyruvat) wurde zusammen mit einem 5 g eines Raney-Ni-Katalysators in einem Autoklaven vorgelegt. Der Autoklav wurde verschlossen, und es wurde NH₃ (66 g; 3,88 mol) kalt aufgepresst. Der Druck wurde mit Wasserstoff auf 20 bar erhöht. Anschließend wurde der Autoklav auf 100°C erhitzt. Der Druck wurde mit Wasserstoff auf 100 bar erhöht, und die Reaktionsmischung wurde unter diesen Bedingung über einen Zeitraum von 3 Stunden gerührt. Danach wurde der Autoklav auf Raumtemperatur abgekühlt und entspannt. Man filtrierte den Katalysator aus dem Reaktionsgemisch ab und analysierte das Filtrat durch NMR-Spektroskopie. Es enthielt als Hauptprodukt das Natrium-Salz des racemischen Alanins. Dieses ließ sich einfach isolieren.
II. Vergleichsbeispiel: Versuch zur Herstellung von racemischem Alanin aus Brenztraubensäure

Beispiel I wurde wiederholt, jedoch setzte man 50 g einer 60 Gew.-% wässrigen Lösung von Brenztraubensäure ein. Man erhielt ein Produktgemisch, das zahlreiche Zersetzungprodukte enthielt und sich durch einfaches Umkristallisieren weder auftrennen noch aufreinigen ließ.

## Patentansprüche

1. Verfahren zur Herstellung von Alkalimetallsalzen von racemischen α-Aminosäuren der allgemeinen Formel (I)
R¹-CH(NH₂)-COOH (I)
in denen R¹ gewählt wird aus Wasserstoff, CH₃, C₂H₅, CH(CH₃)₂, (CH₂)₂COOH und CH₂OH,
**dadurch gekennzeichnet, dass** man ein Alkalimetallsalz der korrespondierenden α-Ketocarbonsäure bzw. Glyoxalsäure in Gegenwart von mindestens einem heterogenen Katalysator, der mindestens ein Übergangsmetall enthält, in Gegenwart von Wasserstoff mit mindestens einer Stickstoffverbindung bei Temperaturen im Bereich von 50 bis 200°C umsetzt, wobei man Stickstoffverbindung wählt aus primären und sekundären Aminen und Ammoniak.

2. Verfahren nach Anspruch 1, **dadurch gekennzeichnet, dass** man heterogene Katalysatoren wählt aus Raney-Metallen und auf einem festen Träger aufgebrachtem Übergangsmetall.

3. Verfahren nach Anspruch 1 oder 2, **dadurch gekennzeichnet, dass** man Übergangsmetall wählt aus Pd, Pt, Ni, Cu und Co.

4. Verfahren nach einem der Ansprüche 1 bis 3, **dadurch gekennzeichnet, dass** Übergangsmetall in heterogenem Katalysator während des Verfahrens zumindest zeitweise in der Oxidationsstufe null vorliegt.

5. Verfahren nach einem der Ansprüche 1 bis 4, **dadurch gekennzeichnet, dass** man Stickstoffverbindung wählt aus Ammoniak.

6. Verfahren nach einem der Ansprüche 1 bis 5, **dadurch gekennzeichnet, dass** man α-Ketocarbonsäure wählt aus Brenztraubensäure.

7. Verfahren nach einem der Ansprüche 1 bis 6, **dadurch gekennzeichnet, dass** man Alkalimetallsalz von α-Ketocarbonsäuren wählt aus Natriumpyruvat.

8. Verfahren nach einem der Ansprüche 1 bis 7, **dadurch gekennzeichnet, dass** man es bei einem Druck im Bereich von Normaldruck bis 300 bar durchführt.

9. Verfahren nach einem der Ansprüche 1 bis 8, **dadurch gekennzeichnet, dass** man es in wässrigem Medium durchführt.

10. Verfahren nach einem der Ansprüche 1 bis 9, **dadurch gekennzeichnet, dass** man zur Aufreinigung des Alkalimetallsalzes der anfallenden racemischen α-Aminosäure oder von Glycin umkristallisiert.

## Claims

1. A process for the preparation of alkali metal salts of racemic α-amino acids of the general formula (I)
R¹-CH(NH₂)-COOH (I)
in which R¹ is selected from hydrogen, CH₃, C₂H₅, CH(CH₃)₂, (CH₂)₂COOH and CH₂OH,
wherein an alkali metal salt of the corresponding α-ketocarboxylic acid or glyoxalic acid is reacted in the presence of at least one heterogeneous catalyst, which comprises at least one transition metal, in the presence of hydrogen with at least one nitrogen compound at temperatures in the range from 50 to 200°C, where the nitrogen compound is selected from primary and secondary amines and ammonia.

2. The process according to claim 1, wherein heterogeneous catalysts are selected from Raney metals and transition metal applied to a solid support.

3. The process according to claim 1 or 2, wherein transition metal is selected from Pd, Pt, Ni, Cu and Co.

4. The process according to any one of claims 1 to 3, wherein transition metal in heterogeneous catalyst is present during the process at least at times in oxidation state zero.

5. The process according to any one of claims 1 to 4, wherein nitrogen compound is selected from ammonia.

6. The process according to any one of claims 1 to 5, wherein α-ketocarboxylic acid is selected from pyruvic acid.

7. The process according to any one of claims 1 to 6, wherein alkali metal salt of α-ketocarboxylic acids is selected from sodium pyruvate.

8. The process according to any one of claims 1 to 7, which is carried out at a pressure in the range from atmospheric pressure to 300 bar.

9. The process according to any one of claims 1 to 8, which is carried out in aqueous medium.

10. The process according to any one of claims 1 to 9, wherein recrystallization is carried out to purify the alkali metal salt of the racemic α-amino acid produced or glycine.

## Revendications

1. Procédé de fabrication de sels de métaux alcalins d'acides α-aminés racémiques de formule générale (I)
R¹-CH(NH₂)-COOH (I)
dans laquelle R¹ est choisi parmi hydrogène, CH₃, C₂H₅, CH(CH₃)₂, (CH₂)₂COOH et CH₂OH,
**caractérisé en ce qu'**un sel de métal alcalin de l'acide α-cétocarboxylique correspondant ou de l'acide glyoxalique est mis en réaction en présence d'au moins un catalyseur hétérogène, qui contient au moins un métal de transition, en présence d'hydrogène, avec au moins un composé d'azote à des températures dans la plage allant de 50 à 200 °C, le composé d'azote étant choisi parmi les amines primaires et secondaires et l'ammoniac.

2. Procédé selon la revendication 1, **caractérisé en ce que** les catalyseurs hétérogènes sont choisis parmi les métaux de Raney et un métal de transition appliqué sur un support solide.

3. Procédé selon la revendication 1 ou 2, **caractérisé en ce que** le métal de transition est choisi parmi Pd, Pt, Ni, Cu et Co.

4. Procédé selon l'une quelconque des revendications 1 à 3, **caractérisé en ce que** le métal de transition dans le catalyseur hétérogène pendant le procédé se présente au moins temporairement au niveau d'oxydation zéro.

5. Procédé selon l'une quelconque des revendications 1 à 4, **caractérisé en ce que** le composé d'azote est choisi parmi l'ammoniac.

6. Procédé selon l'une quelconque des revendications 1 à 5, **caractérisé en ce que** l'acide α-cétocarboxylique est choisi parmi l'acide pyruvique.

7. Procédé selon l'une quelconque des revendications 1 à 6, **caractérisé en ce que** le sel de métal alcalin d'acides α-cétocarboxyliques est choisi parmi le pyruvate de sodium.

8. Procédé selon l'une quelconque des revendications 1 à 7, **caractérisé en ce qu'**il est réalisé à une pression dans la plage allant de la pression normale à 300 bar.

9. Procédé selon l'une quelconque des revendications 1 à 8, **caractérisé en ce qu'**il est réalisé en milieu aqueux.

10. Procédé selon l'une quelconque des revendications 1 à 9, **caractérisé en ce qu'**une recristallisation est effectuée pour la purification du sel de métal alcalin de l'acide α-aminé racémique formé ou de la glycine.
